# EUROPEAN PATENT SPECIFICATION

(11) **EP 4 141 114 B1**
(45) Date of publication and mention of the grant of the patent: **30.09.2026**
(21) Application number: 21792472.9
(22) Date of filing: 23.04.2021
(51) Int. Cl.: C12Q 1/6883, C12N 15/09, C12Q 1/6874, G01N 33/50

(54) **METHOD FOR DETECTING SEVERITY OF PREMENSTRUAL SYNDROME**
VERFAHREN ZUR ERKENNUNG DER SCHWERE DES PRÄMENSTRUELLEN SYNDROMS
PROCÉDÉ DE DÉTECTION DE GRAVITÉ DU SYNDROME PRÉMENSTRUEL

(30) Priority: 24.04.2020 JP 2020077036
(43) Date of publication of application: 01.03.2023
(73) Proprietor: Kao Corporation, Chuo-ku Tokyo 103-8210 (JP)
(72) Inventor: NAGAMORI, Natsumi, Haga-gun, Tochigi 321-3497 (JP); INOUE, Takayoshi, Haga-gun, Tochigi 321-3497 (JP)
(74) Representative: Vossius & Partner Patentanwälte Rechtsanwälte mbB
(86) International application number: PCT/JP2021/016470
(87) International publication number: WO 2021/215532

(56) References cited:
- EP-A1- 3 372 693
- JP-A- 2018 000 206
- BERTONE-JOHNSON E. R. ET AL: "Association of inflammation markers with menstrual symptom severity and premenstrual syndrome in young women", HUMAN REPRODUCTION, vol. 29, no. 9, 2 September 2014 (2014-09-02), GB, pages 1987 - 1994, XP055869562, ISSN: 0268-1161, DOI: 10.1093/humrep/deu170
- DUBEY N ET AL: "The ESC/E(Z) complex, an effector of response to ovarian steroids, manifests an intrinsic difference in cells from women with premenstrual dysphoric disorder", MOLECULAR PSYCHIATRY, vol. 22, no. 8, 3 August 2017 (2017-08-03), London, pages 1172 - 1184, XP093144000, ISSN: 1359-4184, [retrieved on 20240326], DOI: 10.1038/mp.2016.229
- MAUSSION GILLES ET AL: "Functional Analysis of microRNA Expression Profiles in Brain Tissue from Suicide Completers", BIOLOGICAL PSYCHIATRY, ELSEVIER, AMSTERDAM, NL, vol. 67, no. 9, suppl, 1 May 2010 (2010-05-01), pages 55s, XP009180792, ISSN: 0006-3223
- ALAM FARZANA ET AL: "Beta-Arrestin1 Levels in Mononuclear Leukocytes Support Depression Scores for Women with Premenstrual Dysphoric Disorder", vol. 13, no. 1, 22 December 2015 (2015-12-22), Switzerland, pages 43, XP093147569, ISSN: 1660-4601, Retrieved from the Internet <URL:https://www.ncbi.nlm.nih.gov/pmc/articles/PMC4730434/pdf/ijerph-13-00043.pdf> DOI: 10.3390/ijerph13010043
- BAHRAMI AFSANE ET AL: "Menstrual problems in adolescence: relationship to serum vitamins A and E, and systemic inflammation", ARCHIVES OF GYNECOLOGY AND OBSTETRICS, SPRINGER BERLIN HEIDELBERG, BERLIN/HEIDELBERG, vol. 301, no. 1, 16 November 2019 (2019-11-16), pages 189 - 197, XP037036182, ISSN: 0932-0067, [retrieved on 20191116], DOI: 10.1007/S00404-019-05343-1
- DATABASE Nucleotide [online] 15 July 2006 (2006-07-15), "Homo sapiens SH3 domain binding glutamic acid- rich protein like 3, mRNA (cDNA clone MGC: 19619 IMAGE:4040656", XP055868305, retrieved from NCBI Database accession no. BC030135.2
- DATABASE Nucleotide [online] 7 October 2008 (2008-10-07), "Homo sapiens mRNA for NADH-ubiquinone oxidoreductase subunit B14.7 (NDUFA11 gene", XP055869545, retrieved from NCBI Database accession no. AJ539081.1
- DATABASE Nucleotide 16 October 2008 (2008-10-16), "Homo sapiens full open reading frame cDNA clone RZPDo834D0927D for gene PSME2, proteasome (prosome, macropain) activator subunit 2 (PA28 beta); complete eds, without stopcodon", XP055869554, retrieved from NCBI Database accession no. CR541657.1
- DATABASE Nucleotide [online] 24 June 2018 (2018-06-24), "Homo sapiens C-C motif chemokine ligand 22 (CCL22", XP055869560, retrieved from NCBI Database accession no. NM_002990.4
- BERTONE-JOHNSON E. R., RONNENBERG A. G., HOUGHTON S. C., NOBLES C., ZAGARINS S. E., TAKASHIMA-UEBELHOER B. B., FARAJ J. L., WHITCO: "Association of inflammation markers with menstrual symptom severity and premenstrual syndrome in young women", HUMAN REPRODUCTION, OXFORD JOURNALS, GB, vol. 29, no. 9, 1 September 2014 (2014-09-01), GB , pages 1987 - 1994, XP055869562, ISSN: 0268-1161, DOI: 10.1093/humrep/deu170
- BAHRAMI, A ET AL.: "Menstrual problems in adolescence: relationship to serum vitamins A and E, and systemic inflammation", ARCH. GYNECOL. OBSTET., vol. 301, 16 November 2019 (2019-11-16), pages 189 - 197, XP037036182, DOI: 10.1007/s00404-019-05343-1

## Description

### Field of the Invention

The present invention relates to a method of detecting the severity of premenstrual syndrome.

### Background of the Invention

Physical and mental discomfort in women just before and/or during menstruation is called symptoms associated with menstruation and is represented by premenstrual syndrome (hereinafter, referred to as PMS) and dysmenorrhea. Since it has been indicated that PMS is also related to women's labor productivity, an improvement in quality of life (QOL) of women by relieving or reducing the symptoms associated with menstruation represented by PMS and dysmenorrhea is important not only for health but also for society.

It has been developed techniques for examining human current and, in addition, future in vivo physiological states by analysis of nucleic acids such as DNA and RNA in a biological specimen. The analysis of nucleic acids has an advantage of being capable of obtaining abundant information by a single analysis because a comprehensive analysis method has been established and also an advantage of being capable of easily performing functional association of analysis results based on many research reports on single nucleotide polymorphism, RNA function, and the like. Nucleic acids of a biological origin can be extracted from, for example, a tissue, a body fluid, or a secretion, such as blood. Patent Literature 1 discloses that RNA included in skin surface lipids (SSL) is used as a specimen for biological analysis and that marker genes of epidermis, sweat glands, hair follicles, and sebaceous glands were detected from SSL.

There are genes of which expression changes with production of a female hormone, estrogen or progesterone. For example, AQP3 (aquaporin 3), HIF-1α (hypoxia inducible factor 1 subunit α), MFN2 (mitofusin 2), DEFB4A (defensin beta 4A or hBD-2), and hBD-3 have been reported so far (Non Patent Literatures 1 to 3 and Patent Literature 2).

(Patent Literature 1) WO 2018/008319
(Patent Literature 2) JP-A-2015-228829

(Non Patent Literature 1) Hum. Reprod., 2018, 33(11): 2060-2073
(Non Patent Literature 2) Biol. Reprod., 2018, 99(2): 308-318
(Non Patent Literature 3) Mol. Cell. Endocrinol., 2013, 371: 79-86

BERTONE-JOHNSON E. R. ET AL, HUMAN REPRODUCTION, vol. 29, no. 9, 2 September 2014, pages 1987-1994, relates to the association of inflammation markers with menstrual symptom severity and premenstrual syndrome in young women.

### Summary of the Invention

Present invention is defined by the appended claims. The present disclosure provides a method of detecting the severity of PMS of a subject, the method comprising measuring expression of at least one selected from the group consisting of the following genes: SH3BGRL3, NDUFA11, PSME2, SNORA24, SSR4, ZNF91, SNORA70, HSPA6, ALDH2, BRK1, C20orf111, CSNK1A1, STK10, GHITM, RPL21, MED13, RPL32, UBE2R2, RPS3, PLEKHM2, DDX6, ALDH1A3, TSPYL1, NUB1, KRT6A, CCL22, ELOVL3, TMEM66, GDI2, MYL12B, CCNI, PSMC6, RABGEF1, ARF1, ACTN1, CHMP2A, GAK, STX3, SERP1, and GAS7; and translation products of the genes.

In addition, the present disclosure provides a marker of the severity of PMS, the marker comprising at least one selected from the group consisting of nucleic acids derived from the following genes: SH3BGRL3, NDUFA11, PSME2, SNORA24, SSR4, ZNF91, SNORA70, HSPA6, ALDH2, BRK1, C20orf111, CSNK1A1, STK10, GHITM, RPL21, MED13, RPL32, UBE2R2, RPS3, PLEKHM2, DDX6, ALDH1A3, TSPYL1, NUB1, KRT6A, CCL22, ELOVL3, TMEM66, GDI2, MYL12B, CCNI, PSMC6, RABGEF1, ARF1, ACTN1, CHMP2A, GAK, STX3, SERP1, and GAS7; and translation products of the genes.

### Detailed Description of the Invention

The premenstrual syndrome (PMS) is a physical symptom group or mental symptom group that develops about one week before menstruation (luteal phase), which was reported first by Frank in 1931 (Archives of Neurology and Psychiatry, 1931, 26: 1053). Medically, PMS is defined as "physical and mental symptoms that begin 3 to 10 days before the start of menstruation and decline or disappear with the start of menstruation". Examples of typical symptom of PMS include, as the physical symptom group, lower abdominal pain, lower back pain, tight lower abdomen, headache, stiff shoulders, cold hands and feet, increased appetite, diarrhea, constipation, swelling, pain in the breast, tight breasts, easily getting acne, rough skin, easily getting tired, and getting sleepy and, as the mental symptom group, getting annoyed, angry easily, aggressive, depressed, and lachrymose and increasing anxiety.

Herein, the phrase "PMS is severe" refers to a state in which the above physical symptoms or mental symptoms of PMS are serious, and the phrase "PMS is mild" refers to a state in which the above physical symptoms or mental symptoms of PMS are slight. Conventionally, the severity of symptoms associated with menstruation, such as PMS, has been assessed based on the scores of, for example, Menstrual Distress Questionnaire (MDQ). As the MDQ score is higher in the luteal phase, the severity of PMS is assessed as higher. For example, according to the MDQ described in Psychological measurement scale collection III (Science Company, published in 2001), when the MDQ score in the luteal phase is 66 points or more, PMS is assessed as severe, and when the MDQ score is 48 points or less, the PMS can be assessed as mild.

The present invention relates to a marker of PMS severity allowing detection of the severity of PMS and a method of detecting the severity of PMS using the marker as defined in the claims.

The present invention provides a method of detecting the severity of PMS using a marker of PMS severity including a new marker as defined in the claims. The markers of PMS severity that is used in the present disclosure can be collected from skin surface lipids (SSL) and therefore can be simply and noninvasively acquired. Accordingly, according to the present disclosure, the severity of PMS can be detected without burdening the subject.

Although the severity of PMS had to be assessed based on interviews with a subject in the luteal phase as described above so far, the present invention enables simple and objective detection of the severity based on the expression of a gene or its translation product. In addition, the present invention enables detection of the severity of PMS in advance based on the expression of a gene or its translation product in the follicular phase preceding the luteal phase in which PMS occurs.

In one aspect, the present invention provides a marker of PMS severity, as defined in the claims. As shown in examples described later, the present inventors found that the expression of 51 genes shown in Table 1 below has relevance with the severity of PMS. Furthermore, in these 51 genes, 40 genes shown in Table 2 were genes that have not been reported to be associated with PMS so far. Accordingly, nucleic acids derived from the genes shown in Table 1 and translation products of the genes can be each used as a marker of the severity of PMS. In addition, nucleic acids derived from the genes shown in Table 2 and translation products of the genes are new markers of PMS severity.

**[Table 1]**

| | | | | |
|---|---|---|---|---|
| SH3BGRL3 | NDUFA11 | CTNNB1 | AQP3 | PSME2 |
| SNORA24 | SSR4 | ZNF91 | ANXA11 | SNORA70 |
| HSPA6 | ALDH2 | ARHGDIB | DEFB4A | BRK1 |
| C20orf111 | CSNK1A1 | BIRC3 | S100A7 | STK10 |
| GHITM | SFN | RPL21 | MED13 | RPL32 |
| UBE2R2 | RPS3 | PLEKHM2 | DDX6 | ALDH1A3 |
| TSPYL1 | NUB1 | KRT6A | CCL22 | ELOVL3 |
| TMEM66 | GDI2 | MYL12B | CCNI | PSMC6 |
| HMGN2 | HIF1A | RABGEF1 | ARF1 | ACTN1 |
| MFN2 | CHMP2A | GAK | STX3 | SERP1 |
| GAS7 | | | | |

**[Table 2]**

| No. | Gene | No. | Gene |
|---|---|---|---|
| 1 | SH3BGRL3 | 21 | DDX6 |
| 2 | NDUFA11 | 22 | ALDH1A3 |
| 3 | PSME2 | 23 | TSPYL1 |
| 4 | SNORA24 | 24 | NUB1 |
| 5 | SSR4 | 25 | KRT6A |
| 6 | ZNF91 | 26 | CCL22 |
| 7 | SNORA70 | 27 | ELOVL3 |
| 8 | HSPA6 | 28 | TMEM66 |
| 9 | ALDH2 | 29 | GDI2 |
| 10 | BRK1 | 30 | MYL12B |
| 11 | C20orf111 | 31 | CCNI |
| 12 | CSNK1A1 | 32 | PSMC6 |
| 13 | STK10 | 33 | RABGEF1 |
| 14 | GHITM | 34 | ARF1 |
| 15 | RPL21 | 35 | ACTN1 |
| 16 | MED13 | 36 | CHMP2A |
| 17 | RPL32 | 37 | GAK |
| 18 | UBE2R2 | 38 | STX3 |
| 19 | RPS3 | 39 | SERP1 |
| 20 | PLEKHM2 | 40 | GAS7 |

Names of genes disclosed herein are in accordance with the Official Symbol described in NCBI ([www.ncbi.nlm.nih.gov/]). Herein, the phrase "a nucleic acid derived from a gene" encompasses, for example, mRNA transcribed from the gene, cDNA prepared from the mRNA, and a reaction product (e.g., a PCR product or clone DNA) from the cDNA.

In the present disclosure, the severity of PMS of a subject can be detected based on the expression level of each gene shown in Table 1 or its translation product. In the present disclosure, the expression level of each gene shown in Table 1 or its translation product can be measured by quantitatively determining a nucleic acid derived from the gene or a translation product of the gene. For example, the expression level of the gene or its translation product can be measured by quantitatively determining mRNA transcribed from the gene, cDNA prepared from the mRNA, a reaction product from the cDNA, or a translation product (such as protein) synthesized by translation of the mRNA.

Nucleic acids derived from the genes shown in Table 1 or translation products of the genes can be prepared from a biological specimen collected from a subject, for example, cells, a body fluid, or a secretion, according to an ordinary method. Preferably, the nucleic acid is mRNA prepared from skin surface lipids (SSL) of a subject. The translation product is preferably a translation product prepared from SSL of a subject.

In the present disclosure, the term "detection" of the severity of premenstrual syndrome can be paraphrased in a term such as prediction, inspection, measurement, determination, or assessment support. The term "detection", "prediction", "inspection", "measurement", "determination", or "assessment" of the severity of premenstrual syndrome in the present invention does not include diagnosis of the severity of premenstrual syndrome by a medical doctor.

Herein, the term "skin surface lipids (SSL)" refers to a lipophilic fraction present on the surface of the skin and is also called sebum. In general, SSL mainly includes secretions secreted from exocrine glands, such as sebaceous glands, on the skin and is present on the skin surface in the form of a thin layer covering the skin surface. SSL includes RNA expressed in skin cells (see Patent Literature 1). The term "skin" herein is a generic name of regions including tissues on the body surface, such as epidermis, dermis, hair follicles, sweat glands, sebaceous glands, and other glands, unless otherwise specified.

In collection of SSL from the skin of a subject, any means used for collection or removal of SSL from the skin can be adopted. Preferably, an SSL absorbent material, an SSL adhesive material, or a tool for scraping off SSL from the skin can be used, as described below. The SSL absorbent material and the SSL adhesive material are not particularly limited as long as they have affinity to SSL, and examples thereof include polypropylene and pulp. More detailed examples of the procedure of collecting SSL from the skin include a method of absorbing SSL to a sheet-like material such as oil-blotting paper and an oil-blotting film, a method of adhering SSL to a glass plate, tape, or the like, and a method of scraping off and collecting SSL with a spatula, scraper, or the like. In order to improve the adsorption of SSL, an SSL absorbent material impregnated with a solvent having high lipophilicity in advance may be used. In contrast, if the SSL absorbent material contains a solvent having high hydrophilicity or moisture, adsorption of SSL is prevented. Accordingly, the content of a solvent having high hydrophilicity or moisture is preferably low. It is preferable to use the SSL absorbent material in a dry state. The site of the skin where SSL is collected is not particularly limited, and examples thereof include those at any site of the body such as the head, face, neck, trunk, and limbs, and a site where sebum is abundantly secreted, for example, the facial skin is preferable.

SSL collected from a subject may be stored for a certain period of time. The collected SSL is preferably stored at a low temperature condition as prompt as possible after collection in order to suppress the decomposition of contained RNA as much as possible. The temperature condition for storing the RNA-containing SSL in the present disclosure may be 0°C or less and is preferably from -20°C±20°C to -80°C±20°C, more preferably from -20°C±10°C to -80°C±10°C, further more preferably from -20°C±20°C to -40°C±20°C, further more preferably from -20°C±10°C to -40°C±10°C, further more preferably - 20°C±10°C, and further more preferably -20°C±5°C. The period of time for storing the RNA-containing SSL at the low temperature condition is not particularly limited and is preferably 12 months or less, for example, 6 hours or more and 12 months or less, more preferably 6 months or less, for example, 1 day or more and 6 months or less, further more preferably 3 months or less, for example, 3 days or more and 3 months or less.

In extraction of RNA from collected SSL, a method that is usually used for extraction or purification of RNA from a biological specimen can be used, including a phenol/chloroform method, an AGPC (acid guanidinium thiocyanate-phenol-chloroform extraction) method, a method involving using a column such as TRIzol (registered trademark), RNeasy (registered trademark), or QIAzol (registered trademark), a method involving using specific magnetic particles coated with silica, a method involving using solid phase reversible immobilization magnetic particles, or extraction with a commercially available RNA extraction reagent such as ISOGEN. In extraction of a translation product from SSL, a method that is usually used for extraction or purification of protein from a biological specimen can be used, including a commercially available protein extraction reagent such as QIAzol Lysis Reagent (Qiagen).

The severity of PMS of a subject can be detected by examining the expression of one or more genes shown in Table 1 or their translation products by using a nucleic acid or translation product prepared by the above procedure. Accordingly, in another aspect, the present disclosure provides a method of detecting the severity of the PMS of a subject, including measuring the expression of at least one selected from the group consisting of the genes shown in Table 1 or their translation products.

The subject in the method of detecting the severity of PMS according to the present disclosure (hereinafter, the method of the present disclosure) is, for example, a female of a human or non-human mammal and may be anyone who needs detection of the severity of PMS. The subject is preferably an animal having SSL on the skin and is more preferably a human.

In one embodiment, the method of the present invention includes measuring the expression of at least one gene (hereinafter, also referred to as target gene (group)) selected from the group consisting of the genes shown in Table 1 in a subject. In another embodiment, the method of the present invention includes measuring the expression of at least one (hereinafter, also referred to as target product (group)) selected from the group consisting of translation products of the genes shown in Table 1 in a subject. Alternatively, both the expression of a target gene (group) and the expression of a target product (group) may be measured. Preferably, the expression of a target gene (group) is measured.

Among the genes shown in Table 1, those shown in Table 2 are genes that have not been reported to be associated with PMS so far. Accordingly, in a preferable embodiment of the method, the target gene (group) is at least one selected from the group consisting of the genes shown in Table 2, and the target product (group) is at least one selected from the group consisting of translation products of the genes shown in Table 2. Furthermore, in the method of the present disclosure, a combination of at least one selected from the group consisting of the genes shown in Table 2 or their translation products and at least one selected from the group consisting of other genes (that is, CTNNB1, AQP3, ANXA1, ARHGDIB, DEFB4A, BIRC3, S100A7, SFN, HMGN2, HIF1A, and MFN2 shown in Table 1) or their translation products may be used as a target gene group or a target product group, and the expression thereof may be measured.

Preferably, the measurement of the expression of the target gene (group) or the target product (group) in the method of the present disclosure is performed by using RNA or a translation product included in SSL of a subject. In one embodiment, the method of the present disclosure may further include collecting SSL of a subject. In one embodiment, the method of the present disclosure may further include extracting RNA or a translation product from SSL collected from the subject.

The expression level of a target gene can be measured by quantitatively determining the amount of RNA derived from the target gene included in a specimen derived from a subject, for example, RNA extracted from SSL. The measurement of the amount of RNA derived from a subject may be conducted according to a procedure of gene expression analysis by using RNA which is usually used in the art. Examples of the method of gene expression analysis by using RNA include a method in which RNA is converted into cDNA by reverse transcription and the cDNA or its amplification product is then quantitatively determined by real-time PCR, multiplex PCR, microarray, sequencing, chromatography, or the like. The expression level of a translation product can be measured by a protein quantification method which is usually used in the art, for example, ELISA, immunostaining, a fluorescence method, electrophoresis, chromatography, or mass spectrometry. The expression level to be measured may be an absolute amount of the expression of a target gene (group) or target product (group) in a biological specimen or may be a relative expression level with respect to another reference material or the total expression level of genes or translation products.

In a preferable embodiment of the method of the present disclosure, the subject-derived RNA is converted into cDNA by reverse transcription, the cDNA is then subjected to PCR, and the resulting reaction product is purified. In the reverse transcription, a primer targeting a specific RNA to be analyzed may be used, but a random primer is preferably used for more comprehensive preservation and analysis of nucleic acids. In the reverse transcription, a common reverse transcriptase or reverse transcription reagent kit can be used. Preferably, a reverse transcriptase or reverse transcription reagent kit having high accuracy and efficiency is used, and examples thereof include M-MLV reverse transcriptase and its modification product and a commercially available reverse transcriptase or reverse transcription reagent kit, such as PrimeScript (registered trademark) Reverse Transcriptase series (Takara Bio Inc.) and SuperScript (registered trademark) Reverse Transcriptase series (Thermo Fischer Scientific, Inc.). For example, SuperScript (registered trademark) III Reverse Transcriptase and SuperScript (registered trademark) VILO cDNA Synthesis kit (both are available from Thermo Fischer Scientific, Inc.) are preferably used. In PCR of the resulting cDNA, only a specific DNA may be amplified using a primer pair targeting the specific DNA to be analyzed, but a plurality of DNAs may be amplified using a plurality of primer pairs. The PCR is preferably multiplex PCR. The multiplex PCR is a method in which multiple genetic regions are simultaneously amplified by simultaneously using a plurality of primer pairs in a PCR reaction system. The multiplex PCR can be conducted using a commercially available kit (e.g., Ion AmpliSeq Transcriptome Human Gene Expression Kit, Life Technologies Japan Ltd.).

The yield of a PCR reaction product is more improved by adjusting the reaction conditions for the reverse transcription and PCR, and the precision of analysis using it is eventually more improved. Preferably, in an elongation reaction with the reverse transcription, the temperature is preferably adjusted to 42°C±1°C, more preferably 42°C±0.5°C, and further more preferably 42°C±0.25°C, and at the same time, the reaction time is preferably adjusted to 60 minutes or more and more preferably from 80 to 120 minutes. Preferably, the temperature for the annealing and elongation reaction in the PCR can be appropriately adjusted depending on the primers to be used. For example, when the above-mentioned multiplex PCR kit is used, the temperature is preferably 62°C±1°C, more preferably 62°C±0.5°C, and further more preferably 62°C±0.25°C. Accordingly, in the PCR, the annealing and elongation reaction are preferably performed in one step. The time for the step of the annealing and elongation reaction can be adjusted depending on the size or the like of the DNA to be amplified and is preferably from 14 to 18 minutes. The conditions for the degeneration reaction in the PCR can be adjusted depending on the DNA to be amplified and are preferably at from 95°C to 99°C for from 10 to 60 seconds. Reverse transcription and PCR at the above temperature and time can be performed using a thermal cycler that is commonly used in PCR.

The reaction product obtained by the PCR is preferably purified by size separation of the reaction product. The target PCR reaction product can be separated from the primers and other impurities contained in the PCR reaction solution by the size separation. The size separation of DNA can be performed with, for example, a size separation column, a size separation chip, or magnetic beads that can be used in size separation. Preferable examples of the magnetic beads that can be used in size separation include solid phase reversible immobilization (SPRI) magnetic beads such as Ampure XP.

The purified PCR reaction product may be subjected to further treatment necessary for performing subsequent quantitative analysis. For example, for DNA sequencing, the purified PCR reaction product may be prepared into an appropriate buffer solution, the PCR primer region included in the DNA amplified by PCR may be cleaved, or an adaptor sequence may be further added to the amplified DNA. For example, a library for quantitative analysis can be prepared by preparing the purified PCR reaction product into a buffer solution, subjecting the amplified DNA to PCR primer sequence removal and adaptor ligation, and amplifying the resulting reaction product as needed. These operations can be performed using, for example, 5 × VILO RT Reaction Mix included in SuperScript (registered trademark) VILO cDNA Synthesis kit (Life Technologies Japan Ltd.) and 5 × Ion AmpliSeq HiFi Mix and Ion AmpliSeq Transcriptome Human Gene Expression Core Panel included in Ion AmpliSeq Transcriptome Human Gene Expression Kit (Life Technologies Japan Ltd.) according to the protocol attached to each kit. For sequencing, a next-generation sequencer (e.g., Ion S5/XL system, Life Technologies Japan Ltd.) is preferably used. The RNA expression can be quantitatively determined based on the number of reads (read count) produced by sequencing.

Preferably, in the method of the present disclosure, the severity of PMS of a subject is detected based on the expression level of the target gene (group) or the target product (group). For example, the genes shown in Table 1 can be classified into two groups shown in Tables 3 and 4 according to the pattern of variation in the expression level associated with the severity of PMS. The severity of PMS of a subject can be detected utilizing these patterns of variation.

The genes shown in Table 3 are genes of which the expression increases when PMS is severe and decrease when PMS is mild. In contrast, the genes shown in Table 4 are genes of which the expression decreases when PMS is severe and increase when PMS is mild. The severity (e.g., severe or not) of PMS of a subject can be detected by using the expression level of at least one of the genes shown in Tables 3 and 4 or their translation products as a criterion. Among the genes shown in Tables 3 and 4, underlined genes, 22 and 18 genes respectively, are genes (genes shown in Table 2) that have not been reported to be associated with PMS so far.

**[Table 3]**

| | | | |
|---|---|---|---|
| SH3BGRL3 | NDUFA11 | ZNF91 | ANXA11 |
| HSPA6 | ARHGDIB | C20orf111 | STK10 |
| GHITM | PLEKHM2 | TSPYL1 | ELOVL3 |
| TMEM66 | MYL12B | CCNI | PSMC6 |
| HIF1A | RABGEF1 | ARF1 | ACTN1 |
| MFN2 | CHMP2A | GAK | STX3 |
| SERP1 | GAS7 | | |

**[Table 4]**

| | | | |
|---|---|---|---|
| CTNNB1 | AQP3 | PSME2 | SNORA24 |
| SSR4 | SNORA70 | ALDH2 | DEFB4A |
| BRK1 | CSNK1A1 | BIRC3 | S100A7 |
| SFN | RPL21 | MED13 | RPL32 |
| UBE2R2 | RPS3 | DDX6 | ALDH1A3 |
| NUB1 | KRT6A | CCL22 | GDI2 |
| HMGN2 | | | |

In one embodiment of the method of the present disclosure, the expression level of an individual target gene (group) or target product (group) is directly used as a parameter for detecting the severity of PMS. For example, the severity of PMS of a subject can be detected by measuring the expression level of a target gene or target product of the subject on a regular basis (e.g., every month) in a specific phase of a menstrual cycle (e.g., the follicular phase or luteal phase, the same shall apply hereinafter) and tracing variation in the expression level. Alternatively, the severity of PMS of a subject can be detected by comparing the expression level of a target gene or target product measured for the subject in a specific phase of a menstrual cycle with a standard expression level of the target gene or target product during the same phase of a menstrual cycle. As the standard expression level of a target gene or target product, a previously determined value, for example, a statistic of the expression levels (e.g., average expression level) of the target gene or target product in a specific phase of a menstrual cycle can be used. The standard expression level may be set for each subject. For example, the standard expression level may be calculated from a statistic (e.g., average) of expression levels of the target gene or target product measured multiple times for a single subject in a specific phase of the menstrual cycle. Alternatively, the standard expression level may be a statistic (e.g., average) of expression levels of the target gene or target product measured for a group in a specific phase of the menstrual cycle. When a plurality of genes or translation products are used as the target gene group or target product group, a standard expression level is preferably determined for each of the genes or translation products.

For example, in the case where the target gene or target product is a gene shown in Table 3 or its translation product, it can be detected that PMS of the subject is severe if the expression level is higher than the standard expression level, and it can be detected that PMS of the subject is not severe (or to be mild) if the expression level is lower than the standard expression level. In contrast, in the case where the target gene or target product is a gene shown in Table 4 or its translation product, it can be detected that e PMS of the subject is severe if the expression level is lower than the standard expression level, and it can be detected that PMS of the subject is be not severe (or to be mild) if the expression level is higher than the standard expression level.

Alternatively, a combination of two or more of the genes shown in Tables 3 and 4 or their translation products may be used as the target gene or target product. When a plurality of genes or translation products are used, the severity of PMS of a subject can be detected based on whether a certain proportion, for example, 50% or more, preferably 70% or more, more preferably 90% or more, and further more preferably 100% of the genes or translation products satisfies the above-described criterion of the expression level or not. For example, if the expression level of 50% or more of a target gene group selected from Table 3 or a target product group is higher than the standard expression level, it can be detected that PMS of the subject is severe. Alternatively, if the expression level of 50% or more of a target gene group selected from Table 4 or a target product group is lower than the standard expression level, it can be detected that PMS of the subject is severe. Alternatively, if the expression level of 50% or more of a target gene group selected from Table 3 or a target product group is higher than the standard expression level and the expression level of 50% or more of a target gene group selected from Table 4 or a target product group is lower than the standard expression level, it can be detected that PMS of the subject is severe. However, the combination of a target gene group or target product group in the method of the present disclosure is not limited to the above examples.

In another embodiment, the severity of PMS of a subject is detected based on a prediction model constructed using expression data of a target gene (group) or target product (group). As the expression data, data about the expression level of a target gene (group) or target product (group) in a specific phase of a menstrual cycle (e.g., the follicular phase or the luteal phase) can be used. For example, a prediction model optimum for detection of the severity of PMS can be constructed by machine learning using the expression data about each of one or more target genes (group) or target products (group) as an explanatory variable and using the severity of PMS (e.g., severe or mild) as an objective variable. For example, a plurality of genes or translation products that show a larger difference in expression between severe and mild groups of PMS severity is selected from the genes shown in Table 1 or 2 or their translation products, and the expression data thereof can be each used as the explanatory variable.

Alternatively, when expression data of a plurality of genes or translation products are used in construction of a prediction model, the prediction model may be constructed after compression of the data by dimension reduction as needed. For example, a plurality of genes is selected from the genes shown in Table 1 or 2, and the selected genes or their translation products are extracted as a target gene group or a target product group. Subsequently, the expression level of the extracted target gene group or target product group is subjected to principal component analysis. A prediction model optimum for detection of the severity of PMS can be constructed by machine learning using one or more principal components computed by the principal component analysis as an explanatory variable and using the severity of PMS (e.g., severe or mild) as an objective variable.

As the target gene (group) or target product (group) to be used for construction of a prediction model, one or more genes or their translation products showing a larger difference in expression between severe and mild groups of PMS severity are preferably selected from the genes shown in Table 1 or their translation products. For example, one or more genes selected from the 40 genes shown in Table 2, which are genes that have not been reported to be associated with PMS so far, or their translation products are mentioned as preferable target genes (group) or target products (group) to be used for construction of a prediction model. The same also applies to the target gene (group) or target product (group) to be used for principal component analysis.

In one embodiment, about 5 to 20 genes (e.g., genes of Nos. 1 to 20 in Table 2), preferably about 5 to 15 genes, showing a larger difference in expression between severe and mild groups of PMS in the luteal phase are selected from the genes shown in Table 2. More specifically, for example, 5 genes of Nos. 1 to 5, 10 genes of Nos. 1 to 10, or 15 genes of Nos. 1 to 15, preferably 10 genes of Nos. 1 to 10 or 15 genes of Nos. 1 to 15, are selected from Table 2. A prediction model can be constructed using data about the expression levels of the selected genes in the luteal phase as an explanatory variable. In another embodiment, about 5 to 20 genes (e.g., genes of Nos. 1 to 20 in Table 2), preferably about 5 to 15 genes, showing a larger difference in expression between severe and mild groups of PMS in the luteal phase are selected from the genes shown in Table 2. More specifically, for example, 5 genes of Nos. 1 to 5, 10 genes of Nos. 1 to 10, or 15 genes of Nos. 1 to 15, preferably 5 genes of Nos. 1 to 5, are selected from Table 2. A prediction model can be constructed using data about the expression levels of the selected genes in the follicular phase as an explanatory variable.

When data compression is performed in principal component analysis, the dimension and number of the principal component used in construction of a prediction model are not particularly limited, but it is preferable to use one or more principal components including a component having a high contribution rate, for example, a first principal component and preferably having a total contribution rate of 20% or more, more preferably 50% or more, as an explanatory variable. In one embodiment, a prediction model can be constructed by subjecting the expression data of a target gene group in the luteal phase to principal component analysis, selecting principal components so as to include a first principal component and have a total contribution rate of 20% or more, preferably 50% or more, for example, preferably from 22% to 55% or more, more preferably from 50% to 70%, and further more preferably from 55% to 65%, and using the selected principal components as an explanatory variable. In another embodiment, a prediction model can be constructed by subjecting the expression data of a target gene group in the follicular phase to principal component analysis, selecting principal components so as to include a first principal component and have a total contribution rate of 80% or more, preferably 85% or more, and more preferably 90% or more, and using the selected principal components as an explanatory variable.

As the algorithm of construction of a prediction model, known algorithm used in machine learning, can be utilized. Examples of the machine learning algorithm include a random forest, a neural network, a support vector machine of linear kernel (SVM (linear)), and a support vector machine of rbf kernel (SVM (rbf)). Data for verification are input to the constructed prediction model to compute a prediction value, and a model in which the prediction value most conforms to the measured value, for example, a model having high accuracy which is a value showing the degree of agreement between the prediction value and the measured value can be selected as an optimum prediction model.

The severity of PMS of a subject can be detected by inputting the expression data (or a principal component computed therefrom) of the target gene (group) or target product (group) in the subject to the constructed prediction model.

### EXAMPLES

The present invention will now be described based on Examples in further detail but is not limited thereto.

### Example 1: Identification of PMS severity marker

### 1) Collection of SSL

The subjects were 38 healthy subjects (20 to 45 years old females, BMI: 18.5 or more and less than 25.0). The healthy subjects were confirmed in advance that their menstrual cycles were stable every time and about 25 to 30 days. Sebum was collected from the entire face of each subject with an oil-blotting film (5.0 cm × 8.0 cm, 3M Company) in each phase of a menstrual cycle (menstrual phase: any day from day 2 to day 4 after the start of menstruation, follicular phase: any day from day 10 to day 12 after the start of menstruation, luteal phase: any day from day 21 to day 23 after the start of menstruation). The oil-blotting film was transferred into a glass vial and was stored at -80°C for about one month until used for RNA extraction. In addition, after the collection of sebum, the severity of symptoms associated with menstruation (such as dysmenorrhea and premenstrual syndrome) in each phase of the menstrual cycle (the menstrual phase, the follicular phase, and the luteal phase) of each subject was assessed using the Menstrual Distress Questionnaire (MDQ) described in Psychological measurement scale collection III (Science Company, published in 2001), which is a questionnaire for assessing menstruation-related symptoms.

### 2) RNA preparation and sequencing

The oil-blotting films of the above 1) were each cut into a suitable size, and RNA was extracted using QIAzol Lysis Reagent (Qiagen) according to the attached protocol. The extracted RNA was reverse transcribed under conditions of 42°C for 105 minutes, and the resulting cDNA was amplified by multiplex PCR. The multiplex PCR was conducted at 62°C of annealing and elongation temperature. The resulting PCR product was purified with Ampure XP (Beckman Coulter, Inc.). A solution of the resulting purification product was mixed with 5 × VILO RT Reaction Mix included in SuperScript VILO cDNA Synthesis kit and 5 × Ion AmpliSeq HiFi Mix and Ion AmpliSeq Transcriptome Human Gene Expression Core Panel included in Ion AmpliSeq Transcriptome Human Gene Expression Kit (Life Technologies Japan Ltd.). Subsequently, primer sequence digestion, adapter ligation and purification, and amplification were performed according to the protocol attached to each kit to prepare a library. The prepared library was loaded onto Ion 540 Chip and sequenced using the Ion S5/XL system (Life Technologies Japan Ltd.). Each read sequence obtained by sequencing was subjected to gene mapping to a reference sequence, hg19 AmpliSeq Transcriptome ERCC v1, of the human genome to determine the gene derived from each read sequence.

### 3) Grouping of subjects according to MDQ score

In MDQ, it is indicated that the higher the score, the higher the severity of symptoms associated with menstruation. Accordingly, the 38 subjects were grouped according to the scores of MDQ obtained in the luteal phase, and 15 subjects whose scores were each 66 points or more (average score: 87.4) were grouped into a PMS severe group, and 15 subjects whose scores were each 48 points or less (average score: 40.3) were grouped into a PMS mild group. Regarding 8 subjects who were moderate were excluded from the following data analysis.

### 4) Data analysis

The read count of each read obtained by sequencing of RNA derived from SSL of the subjects in the follicular phase and the luteal phase acquired in the above 2) was used as the data of the expression level of each RNA, and reads with a read count of less than 10 were treated as missing values. In order to correct the difference in the total read count between samples, each read count was converted into an RPM (Reads per million mapped reads) value. Selected were 1884 genes that gave expression level data that are not missing values in 80% or more of the subjects. These 1884 genes were subjected to a Student's t-test between groups (PMS severe group and mild group) based on their expression level data (RPM values) in the luteal phase in which PMS develops, and genes with p < 0.05 were extracted. The missing value was supplemented with Singular Value Decomposition (SVD) imputation. As genes of which expression varies between the groups, 51 genes were acquired (Table 5). Among them, 40 genes shown in Table 6 were genes that have not been reported to be associated with PMS so far. Nucleic acids derived from these genes in Table 5 and translation products of the genes can be each used as a marker of the severity of PMS. Furthermore, among these genes in Table 5, nucleic acids derived from the genes shown in Table 6 and translation products of the genes are new markers of the severity of PMS.

**[Table 5]**

| No. | Gene | Regulation | p value | No. | Gene | Regulation | p value |
|---|---|---|---|---|---|---|---|
| 1 | SH3BGRL 3 | UP | 0.004119 | 27 | RPS3 | DOWN | 0.025994 |
| 2 | NDUFA11 | UP | 0.005118 | 28 | PLEKHM2 | UP | 0.026991 |
| 3 | CTNNB1 | DOWN | 0.006184 | 29 | DDX6 | DOWN | 0.027268 |
| 4 | AQP3 | DOWN | 0.006632 | 30 | ALDH1A3 | DOWN | 0.028954 |
| 5 | PSME2 | DOWN | 0.006832 | 31 | TSPYL1 | UP | 0.029321 |
| 6 | SNORA24 | DOWN | 0.007349 | 32 | NUB1 | DOWN | 0.029654 |
| 7 | SSR4 | DOWN | 0.008665 | 33 | KRT6A | DOWN | 0.029955 |
| 8 | ZNF91 | UP | 0.01035 | 34 | CCL22 | DOWN | 0.030458 |
| 9 | ANXA11 | UP | 0.011252 | 35 | ELOVL3 | UP | 0.031205 |
| 10 | SNORA70 | DOWN | 0.011461 | 36 | TMEM66 | UP | 0.032555 |
| 11 | HSPA6 | UP | 0.012576 | 37 | GDI2 | DOWN | 0.03406 |
| 12 | ALDH2 | DOWN | 0.014173 | 38 | MYL12B | UP | 0.03756 |
| 13 | ARHGDIB | UP | 0.014218 | 39 | CCNI | UP | 0.037949 |
| 14 | DEFB4A | DOWN | 0.014951 | 40 | PSMC6 | UP | 0.038989 |
| 15 | BRK1 | DOWN | 0.015211 | 41 | HMGN2 | DOWN | 0.040134 |
| 16 | C20orf111 | UP | 0.015757 | 42 | HIF1A | UP | 0.040339 |
| 17 | CSNK1A1 | DOWN | 0.015871 | 43 | RABGEF1 | UP | 0.040631 |
| 18 | BIRC3 | DOWN | 0.017332 | 44 | ARF1 | UP | 0.045145 |
| 19 | S100A7 | DOWN | 0.017732 | 45 | ACTN1 | UP | 0.045616 |
| 20 | STK10 | UP | 0.018515 | 46 | MFN2 | UP | 0.046243 |
| 21 | GHITM | UP | 0.018562 | 47 | CHMP2A | UP | 0.04649 |
| 22 | SFN | DOWN | 0.019512 | 48 | GAK | UP | 0.04688 |
| 23 | RPL21 | DOWN | 0.023274 | 49 | STX3 | UP | 0.047418 |
| 24 | MED13 | DOWN | 0.023594 | 50 | SERP1 | UP | 0.048733 |
| 25 | RPL32 | DOWN | 0.025027 | 51 | GAS7 | UP | 0.048799 |
| 26 | UBE2R2 | DOWN | 0.025235 | | | | |

**[Table 6]**

| No | Gene | Regulation | p value | No. | Gene | Regulation | p value |
|---|---|---|---|---|---|---|---|
| 1 | SH3BGRL3 | UP | 0.004119 | 21 | DDX6 | DOWN | 0.027268 |
| 2 | NDUFA11 | UP | 0.005118 | 22 | ALDH1A3 | DOWN | 0.028954 |
| 3 | PSME2 | DOWN | 0.006832 | 23 | TSPYL1 | UP | 0.029321 |
| 4 | SNORA24 | DOWN | 0.007349 | 24 | NUB1 | DOWN | 0.029654 |
| 5 | SSR4 | DOWN | 0.008665 | 25 | KRT6A | DOWN | 0.029955 |
| 6 | ZNF91 | UP | 0.01035 | 26 | CCL22 | DOWN | 0.030458 |
| 7 | SNORA70 | DOWN | 0.011461 | 27 | ELOVL3 | UP | 0.031205 |
| 8 | HSPA6 | UP | 0.012576 | 28 | TMEM66 | UP | 0.032555 |
| 9 | ALDH2 | DOWN | 0.014173 | 29 | GDI2 | DOWN | 0.03406 |
| 10 | BRK1 | DOWN | 0.015211 | 30 | MYL12B | UP | 0.03756 |
| 11 | C20orf111 | UP | 0.015757 | 31 | CCNI | UP | 0.037949 |
| 12 | CSNK1A1 | DOWN | 0.015871 | 32 | PSMC6 | UP | 0.038989 |
| 13 | STK10 | UP | 0.018515 | 33 | RABGEF1 | UP | 0.040631 |
| 14 | GHITM | UP | 0.018562 | 34 | ARF1 | UP | 0.045145 |
| 15 | RPL21 | DOWN | 0.023274 | 35 | ACTN1 | UP | 0.045616 |
| 16 | MED13 | DOWN | 0.023594 | 36 | CHMP2A | UP | 0.04649 |
| 17 | RPL32 | DOWN | 0.025027 | 37 | GAK | UP | 0.04688 |
| 18 | UBE2R2 | DOWN | 0.025235 | 38 | STX3 | UP | 0.047418 |
| 19 | RPS3 | DOWN | 0.025994 | 39 | SERP1 | UP | 0.048733 |
| 20 | PLEKHM2 | UP | 0.026991 | 40 | GAS7 | UP | 0.048799 |

### Example 2: Construction of PMS severity prediction model based on luteal phase RNA expression level data

### 1) Dataset partitioning

In expression level dataset of RNA derived from SSL of the subjects in the luteal phase obtained in Example 1, the data of 10 subjects in each of the groups (PMS severe group and mild group) corresponding to 67% of the total number of the subjects to be analyzed were used as training data of a PMS severity prediction model, and the data of 5 subjects in each of the groups corresponding to the residual 33% were used as test data to be used for assessment of model precision. In order to approximate the RPM value following a negative binomial distribution to a normal distribution, the expression level data (RPM value of read count) of RNA determined in Example 1 were converted into RPM value converted into a value of logarithm to base 2 (Log₂ RPM value).

### 2) Selection of feature amount

Feature amounts to be used in construction of a prediction model were selected from 40 genes shown in Table 6, that have not been reported to be associated with PMS, among the genes extracted in Example 1 by the following two methods.
(Method 1) Among the genes shown in Table 6, expression level data (Log₂ RPM value) of 5 to 15 genes in which the p-values of the differences in expression between PMS severe and mild groups are smaller, specifically, 5 genes of Nos. 1 to 5 in Table 6, 10 genes of Nos. 1 to 10 in Table 6, or 15 genes of Nos. 1 to 15 in Table 6, were selected as feature amounts for PMS severity detection.
(Method 2) The expression level data of the genes shown in Table 6 were subjected to principal component analysis using the tidyverse package of statistical analysis environment R, and variable compression was conducted. The data (first to tenth principal components, Table 7) obtained by the principal component analysis and having a new dimension were selected as feature amounts for PMS severity detection.

**[Table 7]**

| Principal component | Contribution rate | Accumulated contribution rate |
|---|---|---|
| Principal component 1 | 25.22% | 25.22% |
| Principal component 2 | 10.70% | 35.92% |
| Principal component 3 | 8.71% | 44.63% |
| Principal component 4 | 6.71% | 51.34% |
| Principal component 5 | 5.60% | 56.94% |
| Principal component 6 | 5.42% | 62.36% |
| Principal component 7 | 4.97% | 67.33% |
| Principal component 8 | 4.12% | 71.45% |
| Principal component 9 | 3.66% | 75.11% |
| Principal component 10 | 3.40% | 78.50% |

### 3) Model construction

The model construction was performed using the carnet package of statistical analysis environment R. A prediction model was constructed using the feature amounts selected by the methods 1 and 2 in the above 2) as an explanatory variable and using the PMS severity (severe or mild) as an objective variable. The prediction model was trained by performing 10-fold cross validation for each feature amount using four algorithms: a random forest, a neural network, a support vector machine of linear kernel (SVM (linear)), and a support vector machine of rbf kernel (SVM (rbf)). Regarding each algorithm, the prediction value of PMS severity was calculated by inputting the expression level data (Log₂ RPM value) or principal component data of RNA of the test data to the model after the training. The accuracy, which is a value showing the degree of agreement between the prediction value and the measured value, was calculated, and a model with the highest accuracy value was selected as an optimum prediction model.

### 4) Results

Table 8 shows feature amounts used in detection of PMS severity, optimum algorithms giving maximum accuracy, and the accuracy. PMS severity could be detected with high accuracy based on the expression level data of genes relating to PMS severity in the luteal phase or their principal components. Accordingly, it was demonstrated that PMS severity can be detected using the data of expression level of SSL-derived RNA in the luteal phase. In addition, in the case of prediction models based on principal components, the accuracy of the prediction model constructed by the first to fifth principal components was higher than that of the prediction model constructed by principal component 1 and was equal to that of the prediction model constructed by the first to sixth principal components. These results demonstrated that a PMS severity prediction model with higher precision can be constructed by using one or more principal components giving a total contribution rate of 20% or more, for example, from 22% to 55% or more, preferably from 50% to 70%, and more preferably from 55% to 65%, as an explanatory variable. In the case of prediction models constructed directly from gene expression levels, the accuracy of the prediction model constructed by 10 genes was higher than that of the prediction model constructed by 5 genes and was equal to that of the prediction model constructed by 15 genes. It was demonstrated that a PMS severity prediction model with higher precision can be constructed by using the expression data of about 10 to 15 or more genes with larger differences in expression due to PMS severity.

**[Table 8]**

| Detection item | Feature amount used | Optimum algorithm | Accuracy (%) |
|---|---|---|---|
| PMS severity | 5 genes of Nos. 1 to 5 in Table 6 | SVM (rbf) | 70 |
| PMS severity | 10 genes of Nos. 1 to 10 in Table 6 | SVM (rbf) | 90 |
| PMS severity | 15 genes of Nos. 1 to 15 in Table 6 | SVM (rbf) | 90 |
| PMS severity | 1st Principal component | SVM (rbf) | 80 |
| PMS severity | 1st to 5th Principal components | SVM (rbf) | 90 |
| PMS severity | 1st to 6th Principal components | Neural net | 90 |

### Example 3: Construction of PMS severity prediction model based on RNA expression level data in the follicular phase

### 1) Dataset partitioning and selection of feature amount

Training data and test data were produced as in the procedure of 1) of Example 2 except that, regarding the genes shown in Table 6, expression level dataset of RNA derived from SSL of the subjects in the follicular phase was used. Subsequently, according to the same procedure as in 2) of Example 2, the Log₂ RPM value of the expression level data of RNA and the first to fifteenth principal components (Table 9) obtained by principal component analysis of the expression level data were selected as feature amounts for PMS severity detection.

**[Table 9]**

| Principal component | Contribution rate | Accumulated contribution rate |
|---|---|---|
| Principal component 1 | 25.22% | 25.22% |
| Principal component 2 | 10.70% | 35.92% |
| Principal component 3 | 8.71% | 44.63% |
| Principal component 4 | 6.71% | 51.34% |
| Principal component 5 | 5.60% | 56.94% |
| Principal component 6 | 5.42% | 62.36% |
| Principal component 7 | 4.97% | 67.33% |
| Principal component 8 | 4.12% | 71.45% |
| Principal component 9 | 3.66% | 75.11% |
| Principal component 10 | 3.40% | 78.50% |
| Principal component 11 | 3.15% | 81.66% |
| Principal component 12 | 2.76% | 84.42% |
| Principal component 13 | 2.40% | 86.82% |
| Principal component 14 | 2.17% | 88.99% |
| Principal component 15 | 2.14% | 91.13% |

### 2) Model construction

The feature amounts selected in 1) were used as the explanatory variable, and the prediction model was trained by the same procedure as in 3) of Example 2, and a model with the highest accuracy was selected as an optimum prediction model.

### 3) Results

Table 10 shows feature amounts used in detection of PMS severity, optimum algorithms giving maximum accuracy, and the accuracy. PMS severity could be detected with high accuracy based on the expression level data of genes relating to PMS severity in the follicular phase or their principal components. Accordingly, it was demonstrated that the severity of PMS associated with the subsequent menstruation can be detected using the expression level data of RNA derived from SSL in the follicular phase. In addition, in the case of prediction models based on principal components, it was demonstrated that the accuracy of the prediction model constructed by the first to fifteenth principal components was the highest and that a PMS severity prediction model with higher precision can be constructed by using one or more principal components giving a total contribution rate of 80% or more, preferably 85% or more, and more preferably 90% or more, as an explanatory variable. In the case of prediction models constructed directly from gene expression levels, it was demonstrated that a PMS severity prediction model with higher precision can be constructed by using expression data of about 5 to 20 genes, preferably about 5 to 15 genes, having higher correlation with PMS severity.

**[Table 10]**

| Detection item | Feature amount used | Optimum algorithm | Accuracy (%) |
|---|---|---|---|
| PMS severity | 5 genes of Nos. 1 to 5 in Table 6 | Random Forest | 70 |
| PMS severity | 10 genes of Nos. 1 to 10 in Table 6 | Neural net | 60 |
| PMS severity | 15 genes of Nos. 1 to 15 in Table 6 | Neural net | 60 |
| PMS severity | 1st to 5th Principal components | Neural net | 50 |
| PMS severity | 1st to 10th Principal components | Neural net | 50 |
| PMS severity | 1st to 15th Principal components | Neural net | 80 |

## Claims

1. An in vitro method of detecting severity of premenstrual syndrome of a subject, comprising measuring expression of the SH3BGRL3 gene and/or translation products of the SH3BGRL3 gene.

2. The method according to Claim 1, further comprising measuring expression of at least one selected from the group consisting of following genes: CTNNB1, AQP3, ANXA1, ARHGDIB, DEFB4A, BIRC3, S100A7, SFN, HMGN2, HIF1A, and MFN2, and translation products of the genes.

3. The method according to Claim 1 or 2, comprising detecting severity of premenstrual syndrome based on an expression level of the gene or translation product.

4. The method according to Claim 3, comprising detecting severity of premenstrual syndrome based on an expression level of the gene or translation product in luteal phase or follicular phase.

5. The method according to any one of Claims 1 to 4, comprising:
detecting severity of premenstrual syndrome using a prediction model based on an expression level of the gene or translation product, wherein
the prediction model is constructed using expression levels of the gene or translation product in a severe group and a mild group of premenstrual syndrome or at least one principal component computed by principal component analysis of the expression levels as an explanatory variable and using severity of premenstrual syndrome as an objective variable.

6. The method according to any one of Claims 1 to 5, wherein the expression level of the gene is measured by quantitatively determining mRNA of the gene included in skin surface lipids of the subject.

7. A use of nucleic acids derived from the SH3BGRL3 gene and/or translation products of the SH3BGRL3 gene as a marker of severity of premenstrual syndrome.

8. The use according to Claim 7, wherein the nucleic acid derived from the gene is mRNA of the gene included in skin surface lipids.

## Patentansprüche

1. *In vitro*-Verfahren zum Nachweisen des Schweregrads von Prämenstruellem Syndrom bei einem Individuum, wobei das Verfahren das Messen der Expression des SH3BGRL3-Gens und/oder Translationsprodukten des SH3BGRL3-Gens umfasst.

2. Verfahren nach Anspruch 1, das des Weiteren das Messen der Expression mindestens eines ausgewählt aus der Gruppe bestehend aus den folgenden Genen: CTNNB1, AQP3, ANXA1, ARHGDIB, DEFB4A, BIRC3, S100A7, SFN, HMGN2, HIF1A und MFN2, und Translationsprodukten der Gene umfasst.

3. Verfahren nach Anspruch 1 oder 2, das das Nachweisen des Schweregrads von Prämenstruellem Syndrom basierend auf einem Expressionsspiegel des Gens oder des Translationsprodukts umfasst.

4. Verfahren nach Anspruch 3, das das Nachweisen des Schweregrads von Prämenstruellem Syndrom basierend auf einem Expressionsspiegel des Gens oder des Translationsprodukts in der Lutealphase oder Follikelphase umfasst.

5. Verfahren nach einem der Ansprüche 1 bis 4, das umfasst:
Nachweisen des Schweregrads von Prämenstruellem Syndrom unter Verwendung eines Vorhersagemodells basierend auf einem Expressionsspiegel des Gens oder des Translationsprodukts, wobei das Vorhersagemodell erstellt wird unter Verwendung der Expressionsspiegel des Gens oder des Translationsprodukts bei einer Gruppe mit schweren Symptomen und einer Gruppe mit leichten Symptomen von Prämenstruellem Syndrom oder mindestens einer Hauptkomponente, die mittels Hauptkomponentenanalyse der Expressionsspiegel als erklärende Variable und unter Verwendung des Schweregrads von Prämenstruellem Syndrom als Zielvariable berechnet wird.

6. Verfahren nach einem der Ansprüche 1 bis 5, wobei der Expressionsspiegel des Gens durch quantitatives Bestimmen der mRNA des in Hautoberflächenlipiden enthaltenen Gens des Individuums gemessen wird.

7. Verwendung von Nucleinsäuren, die von dem SH3BGRL3-Gen und/oder den Translationsprodukten des SH3BGRL3-Gens stammen, als Marker für den Schweregrad von Prämenstruellem Syndrom.

8. Verwendung nach Anspruch 7, wobei die von dem Gen stammende Nucleinsäure mRNA des in Hautoberflächenlipiden enthaltenen Gens ist.

## Revendications

1. Méthode in vitro de détection de la sévérité du syndrome prémenstruel chez un sujet, comprenant la mesure de l'expression du gène SH3BGRL3 et/ou de produits de traduction du gène SH3BGRL3.

2. Méthode selon la revendication 1, comprenant en outre la mesure de l'expression d'au moins l'un choisi dans le groupe constitué par les gènes suivants : CTNNB1, AQP3, ANXA1, ARHGDIB, DEFB4A, BIRC3, S100A7, SFN, HMGN2, HIF1A, et MFN2, et les produits de traduction des gènes.

3. Méthode selon la revendication 1 ou 2, comprenant la détection de la sévérité du syndrome prémenstruel sur la base du niveau d'expression du gène ou du produit de traduction.

4. Méthode selon la revendication 3, comprenant la détection de la sévérité du syndrome prémenstruel sur la base du niveau d'expression du gène ou du produit de traduction dans la phase lutéale ou la phase folliculaire.

5. Méthode selon l'une quelconque des revendications 1 à 4, comprenant :
la détection de la sévérité du syndrome prémenstruel utilisant un modèle de prédiction basé sur le niveau d'expression du gène ou du produit de traduction, dans laquelle
le modèle de prédiction est construit par utilisation des niveaux d'expression du gène ou du produit de traduction dans un groupe sévère et un groupe léger de syndrome prémenstruel ou d'au moins une composante principale calculée par analyse des composantes principales des niveaux d'expression sous la forme d'une variable explicative, et utilisation de la sévérité du syndrome prémenstruel en tant que variable expliquée.

6. Méthode selon l'une quelconque des revendications 1 à 5, dans laquelle le niveau d'expression du gène est mesuré par détermination quantitative de l'ARNm du gène inclus dans les lipides de surface de la peau du sujet.

7. Utilisation d'acides nucléiques dérivés du gène SH3BGRL3 et/ou de produits de traduction du gène SH3BGRL3 en tant que marqueurs de sévérité du syndrome prémenstruel.

8. Utilisation selon la revendication 7, dans laquelle l'acide nucléique dérivé du gène est l'ARNm du gène inclus dans les lipides de surface de la peau.
